(19) Europäisches Patentamt
European Patent Office
Office européen des brevets

(11) Veröffentlichungsnummer : **0 386 700 B1**

# (12) EUROPÄISCHE PATENTSCHRIFT

(45) Veröffentlichungstag der Patentschrift : **02.12.92 Patentblatt 92/49**

(51) Int. Cl.$^5$ : **A61K 31/635,** A61K 9/72

(21) Anmeldenummer : **90104270.5**

(22) Anmeldetag : **06.03.90**

## (54) Die Anwendung inhalierter Schleifendiuretika zur Behandlung von allergen-induzierten nasalen Reaktionen.

(30) Priorität : **08.03.89 DE 3907414**

(43) Veröffentlichungstag der Anmeldung : **12.09.90 Patentblatt 90/37**

(45) Bekanntmachung des Hinweises auf die Patenterteilung : **02.12.92 Patentblatt 92/49**

(84) Benannte Vertragsstaaten : **AT BE CH DE DK ES FR GB GR IT LI LU NL SE**

(56) Entgegenhaltungen : **FR-M- 3 500**

(56) Entgegenhaltungen : **THE LANCET, 30 Juli 1988, Seiten 252-255; S. BIANCO et al.: "Prevention of exercise-induced bronchoconstriction by inhaledfrusemide"**
**AM. REV. OF RESPIR. DIS., Band 137, Nr. 4(2) April 1988;, S. BIANCO et al.: "Inhaled furosenide (F) prevents allergen inducedbronchoconstriction in atopic asthmatic"**

(73) Patentinhaber : **HOECHST AKTIENGESELLSCHAFT**
**Postfach 80 03 20**
**W-6230 Frankfurt am Main 80 (DE)**

(72) Erfinder : **Bianco, Sebastiano**
**Via Cottolengo 42**
**I-20143 Milano (IT)**

**EP 0 386 700 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung eines Schleifendiuretikums zur Herstellung einer zur Verabreichnung in die Nase bestimmten Verneblerlösung zur Behandlung allergen-induzierter nasaler Reaktionen.

Schleifendiuretika sind bewährte Diuretika, deren bekannter Vertreter Furosemid mit der Formel

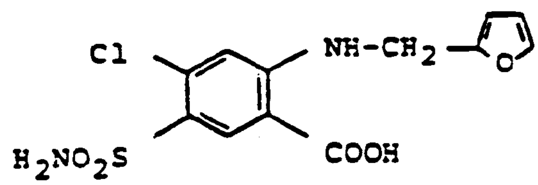

ebenso eingehend untersucht worden ist wie seine Wirkungen. Der Wirkstoff wird üblicherweise oral verabreicht, kann aber zur Förderung der Diurese auch intravenös injiziert werden.

Bei Furosemid handelt es sich um einen Wirkstoff, mit dem außerdem Asthma verhütet oder behandelt werden kann (s. The Lancet, 30. Juli, 1988, S. 252).

Nirgends findet sich allerdings der Hinweis, daß Schleifendiuretika auch zur Bekämpfung allergischer Reaktionen der Nasenschleimhaut eingesetzt werden können. Zu den Schleifendiuretika zählen u.a. neben Furosemid das Bumetanid, Ethacrynsäure, Etolozin oder Piretanid, Torasemid, Indacrinon und Azosemid.

Um zu untersuchen, ob Furosemid als einer der typischen Vertreter der Schleifendiuretika eine protektive Wirkung gegen die allergischen Reaktionen in der Nasenschleimhaut ausübt, wurde eine randomisierte, placebo-kontrollierte Doppelblind-Crossoverstudie über den Einfluß von inhaliertem Furosemid auf die Reaktion gegenüber der nasalen Allergenprovokation angestellt.

PATIENTEN UND METHODEN

PATIENTEN: Unter den Patienten mit einer positiven Reaktion auf die spezifische nasale Provokation wurden 10 Probanden mit allergischer Rhinitis ausgewählt: 4 Männer und 6 Frauen im Alter zwischen 14 und 35 Jahren (Durchschnittsalter 21,9 $\pm$ 1,9 Jahre). Sie wiesen in der Anamnese sämtlich eine allergische Rhinitis auf, zeigten gegenüber dem relevanten Allergen eine positive kutane Sofortreaktion, waren beschwerdefrei oder hatten sehr leichte respiratorische Symptome, hatten seit mindestens 4 Wochen keine Atemwegsinfektionen gehabt und standen nicht in Behandlung. Vier Patienten waren gegen Pollen allergisch, 6 gegen Dermatophagoide. Die Untersuchung der Patienten mit Pollenallergie erfolgte außerhalb der Pollensaison. Bei einer einleitenden Provokation wurden mittels eines Nasenverneblers, der 80 µl/Stoß liefert, 2 Stöße Allergen (Alpha Base, Dome/Hollister-Stier, Bayropharm, Italia, Mailand, Italien) lokal in eine Nasenöffnung verabreicht. Die nasale Resistenz wurde für jede Nasenöffnung vor der Provokation und nach 5, 10, 15, 20, 30, 45 und 60 Minuten mittels vorderer Rhinometrie (Mod. NR4, Mercury Electronics Scotland Ltd, Glasgow, GB) gemessen. Die nasale Resistenz wurde für jede Nasenöffnung getrennt gemessen, indem die Luftströmung registriert wurde, wenn der Druck auf der kontralateralen Seite den Stichprobenpunkt von 150 Pa erreichte. Die Resistenz wurde anschließend als das Verhältnis zwischen kontralateralem Druck (d.h. 150 Pa) und Strömung (s.: Clement PAR. Committee report on standardization of rhinomanometry. Rhinology 1984:22.151-5) berechnet. Wo erforderlich, wurde die nasale Gesamtresistenz mit Hilfe der Formel

$$(R_1 x R_2)/(R_1 + R_2)$$

berechnet, wobei $R_1$ und $R_2$ den für jede einzelne Nasenöffnung ermittelten Werten entsprechen. Als positiv wurden die Patienten eingestuft, die zu einem beliebigen Zeitpunkt in der stimulierten Nasenöffnung eine Zunahme der Resistenz um mindestens 100% gegenüber dem Ausgangswert aufwiesen.

STUDIENPROTOKOLL

Die Wirkung der Vorbehandlung mit inhaliertem Furosemid auf die nasale allergische Reaktion wurde in einer randomisierten Doppelblind-Crossoverstudie untersucht und mit Placebo verglichen. Bei jedem Patienten wurden innerhalb eines Intervalls von 4-8 Tagen zwei spezifische nasale Provokationen vorgenommen, wobei das gleiche Protokoll und die gleiche Allergendosis wie bei der einleitenden Provokation verwendet wurden. Unmittelbar vor der Verabreichung des Allergens wurden den Patienten in beide Nasenöffnungen 2 Stöße Furosemid 10 mg/ml in NaCl 7,0 mg + NaOH q.s. ad pH 9 + $H_2O$ q.s. ad 1 ml, 295 mOsm/kg, pH 8,36 (®Lasix, Hoechst AG, Frankfurt am Main ) oder das Verdünnungsmittel alleine ohne Furosemid verabfolgt; hierzu wurde

ein Nasenvernebler benutzt, der 120 µl/Stoß abgibt. Die nasale Resistenz wurde vor und nach der Vorbehandlung und 5, 10, 15, 20, 30, 45 und 60 Minuten nach der Provokation gemessen. Um eine objektive Beurteilung der durch die Provokation ausgelösten Rhinorrhoe zu erreichen, wurden nach der Provokation 30 Minuten lang die Nasensekrete in Gaze aufgefangen, die sorgfältig in die Nasenöffnungen eingelegt worden war; die Sekretmenge wurde als das Endgewicht der Gaze abzüglich des ursprünglichen Trockengewichts berechnet. Die nasalen Symptome (Pruritus, nasale Obstruktion, Niesen, Lakrimation) wurden anhand eines willkürlichen objektiven Wertes zwischen 0 (fehlt) bis 3 (Maximum) beurteilt.

DATENANALYSE

Die Daten wurden als absolute Werte oder als die Differenz gegenüber den Ausgangswerten zum Zeitpunkt Null, d.h. nach der Behandlung mit Furosemid oder Placebo und unmittelbar vor der Allergenprovokation angegeben.

Die prozentuale protektive Wirkung wurde für jeden Patienten nach folgender Formel berechnet:

(AUC Furosemid - AUC Placebo) / AUC Placebo x 100,

wobei AUC = Integral unter der Zeitreaktionskurve entspricht, ausgedrückt in absoluten Differenzen gegenüber dem Ausgangswert.

Die Zweiweg-Varianzanalyse und der paired Student's t test wurden zum statistischen Vergleich normal verteilter Variablen herangezogen (Snidercor WG, Cochran WG. Statistical methods, 7th ed. Ames, Iowa University Press, 1980). Für den Vergleich der Symptomenwerte diente der McNemar-Test. Ein p-Wert von 0,05 wurde als signifikant angesehen.

UNTERSUCHUNGSERGEBNISSE

Die mittels vorderer Rhinomanometrie gemessene nasale Gesamtresistenz betrug vor der Provokation 0,39 ± 0,07 kPa.$1^{-1}$.Sek. (M±SE) vor Placebo und 0,38±0,06 vor Furosemid. Die Lokalbehandlung mit Furosemid oder Placebo bewirkte keine signifikante Veränderung dieser Parameter.

Nach Placebo kam es bei sämtlichen Patienten zu einer sofortigen obstruktiven, nasalen Reaktion in der stimulierten Nasenöffnung, die zwischen 30 Sekunden und 20 Minuten nach der Provokation einsetzte, nach 10 bis 30 Minuten ein Maximum erreichte und mehr als 60 Minuten lang anhielt. Die nasale Resistenz wurde im Vergleich zum Ausgangswert nach Placebo zu jedem Zeitpunkt nach der Provokation erhöht, wobei die stärkste Abweichung bei 13,7±2,5 lag. Im Gegensatz dazu wurden in der nicht stimulierten Nasenöffnung (maximale Abweichung 3,9±2,4, Tab. 1 und Abb. 1) keine signifikanten Abweichungen registriert, obwohl in zwei Fällen auch auf dieser Seite eine deutliche Zunahme der Resistenz beobachtet wurde. Es kam bei sämtlichen Patienten zu leichtem Niesen, zur Lakrimation, zum nasalen Pruritus (Abb. 2) und zur Rhinorrhoe (4,15±0,77 g, Abb. 3).

Nach Furosemid war die allergen-induzierte Zunahme der nasalen Resistenz im Vergleich zu Placebo während des Gesamtverlaufs des Experimentes deutlich abgeschwächt (Abb. 1), wobei die maximale Veränderung der Resistenz in der stimulierten Nasenöffnung 3,1 ±1,4 ($p < 0,005$ im Vergleich zum Test nach Placebo, Abb. 4) betrug. Die mittels AUC ermittelte protektive Wirkung von Furosemid im Vergleich zu Placebo betrug 87±30% (Abb. 5), wobei die stärkste Abweichung einem einzigen Patienten zuzuordnen war, der nach Placebo eine schlechte Reaktion aufwies, die durch Furosemid nicht abgeschwächt wurde. Selbst bei den beiden Probanden mit einer kontralateralen Reaktion nach Placebo wurden keine signifikanten Abweichungen in der nicht stimulierten Nasenöffnung (maximale Abweichung 0,6±0,5) beobachtet. Auch die Symptomwerte waren in den meisten Fällen geringer, was insbesondere auf den nasalen Pruritus ($p > 0,05$) und die Lakrimation ($p < 0,005$, Abb. 2) zutraf, und die Nasensekretion war im Vergleich zu Placebo stark vermindert (1,63±0,5 g, $p < 0,001$, Abb. 3).

DISKUSSION

Die Lokalbehandlung mit Furosemid resultiert in einer deutlichen Abnahme der nasalen Obstruktion, Sekretion und Symptome, die durch eine spezifische Allergen-Provokation ausgelöst worden waren. Es handelte sich hierbei um einen spezifischen Effekt, da nach Furosemid oder Placebo alleine keine Veränderungen beobachtet wurden; er wurde zudem praktisch bei allen Patienten registriert.

LEGENDEN ZU DEN ABBILDUNGEN

Abb. 1 - Veränderungen des nasalen Luftwegwiderstandes in der stimulierten Nasenöffnung nach Vorbehandlung mit Furosemid (offene Kreise) oder Placebo (ausgefüllte Kreise). *)$p < 0,005$ im Vergleich zum Ausgangswert.

Abb. 2 - Die durch die nasale Provokation nach der Behandlung mit Furosemid oder Placebo ausgelösten Veränderungen der Symptomwerte.

Abb. 3 - Gesamtabsonderung aus der Nase, aufgefangen in den 60 Minuten nach der Allergen-Provokation nach Placebo (links) oder Furosemid (rechts). *)$p < 0,001$ im Vergleich zu Placebo. Jedes Zeichen entspricht einem Patienten. Kreise mit Fehlerbalken entsprechen dem Mittelwert und Standardfehler der gesamten Gruppe nach jeder Behandlung.

Abb. 4 - Maximale Zunahme der nasalen Resistenz in der stimulierten Nasenöffnung gegenüber dem Ausgangswert nach der Vorbehandlung mit Placebo oder Furosemid. *)$p < 0,005$. Übrige Symbole wie in Abb. 3.

Abb. 5 - Bereich unter der Zeitverlaufskurve der Veränderungen der nasalen Resistenz während der 60 Minuten nach der Provokation und vorausgegangener Behandlung mit Placebo oder Furosemid. Symbole wie in Abb. 3.

TABELLE I

Wirkung von Furosemid oder Placebo auf die Veränderungen der Resistenz der nasalen Luftwege nach Allergen-Provokation.

| Zeit (Min.) | STIMULIERTE SEITE | | NICHT STIMULIERTE SEITE | |
|---|---|---|---|---|
| | Placebo | Furosemid | Placebo | Furosemid |
| Vor der Behandlung[a] | 0.78±0.13[b] | 0.67±0.18 | 0.87±0.21 | 0.96±0.20 |
| 0 | 0.88±0.19 | 0.83±0.30 | 1.15±0.55 | 1.34±0.32 |
| 5 | 5.44±2.20 | 0.94±0.20 | 1.46±0.66 | 1.13±0.29 |
| 10 | 6.51±7.72[cd] | 1.63±0.76 | 4.57±2.98 | 0.93±0.16 |
| 15 | 7.20±1.86[c] | 3.23±1.34 | 3.56±2.94 | 1.07±0.26 |
| 20 | 7.29±2.30[c] | 2.23±0.78 | 2.47±1.81 | 1.04±0.24 |
| 30 | 8.95±2.56[cd] | 3.18±1.31 | 2.33±1.60 | 1.41±0.42 |
| 45 | 6.35±1.54[cd] | 1.46±0.37 | 1.13±0.27 | 1.24±0.41 |
| 60 | 6.87±1.75[cd] | 1.85±0.48 | 1.38±0.43 | 1.27±0.52 |

a) Ausgangsmessung vor der Behandlung mit Placebo oder Furosemid

b) M ± SE

c) $p < 0,05$ im Vergleich zu Zeitpunkt 0

d) $p < 0,05$ im Vergleich zu Placebo zum gleichen Zeitpunkt

## Patentansprüche

1. Verwendung eines Schleifendiuretikums zur Herstellung einer zur Verabreichung in die Nase bestimmten Verneblerlösung zur Behandlung der allergen- induzierten Rhinitis.

2. Verwendung von Furosemid zur Herstellung einer zur Verabreichung in die Nase bestimmten Verneblerlösung zur Behandlung der allergen- induzierten Rhinitis.

## Claims

1. Use of a loop diuretic for the preparation of a nebulizing solution to be administered into the nose for the treatment of allergen-induced rhinitis.

2. Use of furosemide for the preparation of a nebulizing solution to be administered into the nose for the treatment of allergen-induced rhinitis.

## Revendications

1. Usage d'un diurétique dit de l'"anse" pour la production d'une solution à nébuliser dans les narines pour le traitement de la rhinite induite par des allergènes.

2. Usage de furosémide pour la production d'une solution à nébuliser dans les narines pour le traitement de la rhinite induite par des allergènes.

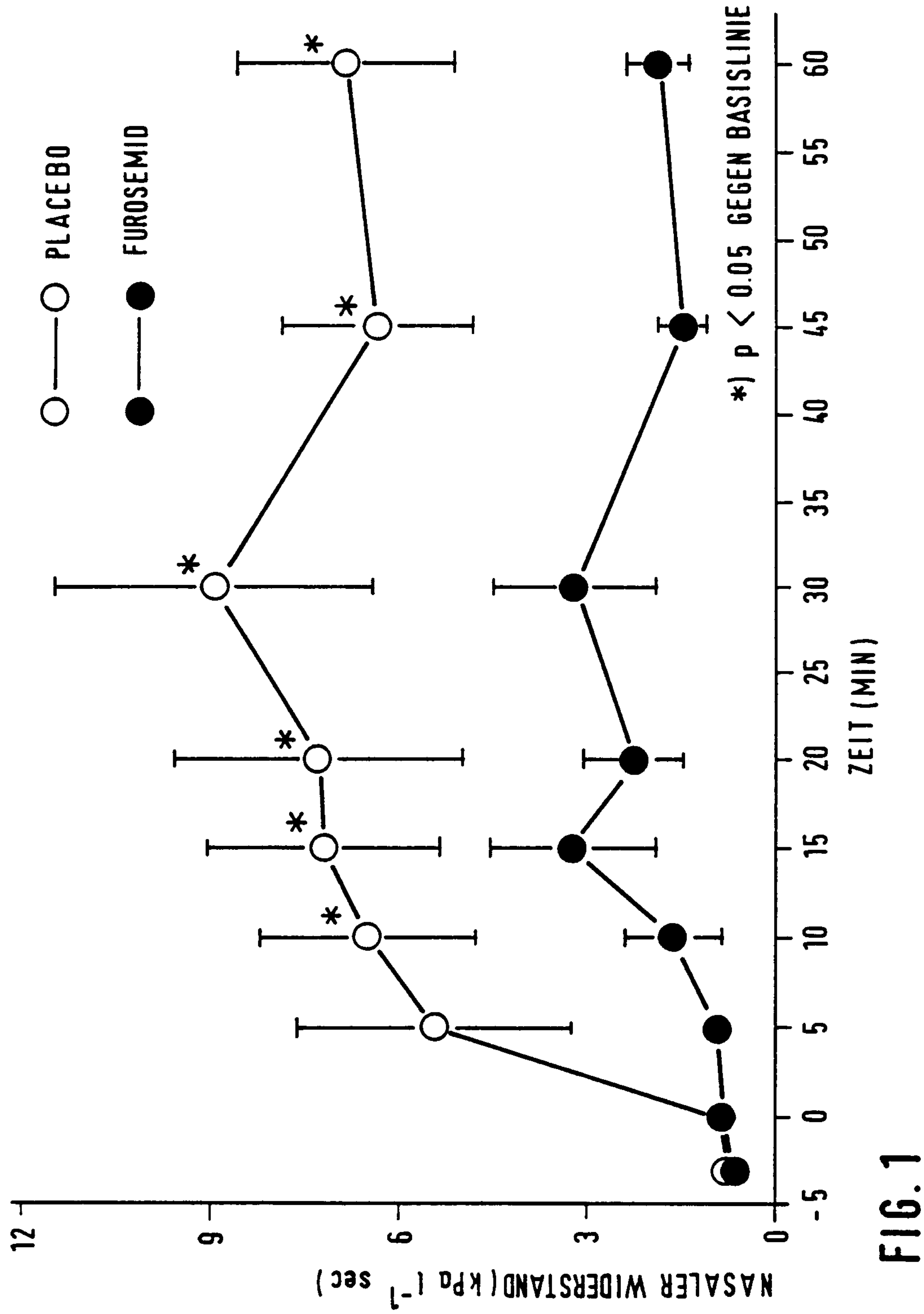
PLACEBO
FUROSEMID
*) p < 0.05 GEGEN BASISLINIE
ZEIT (MIN)
NASALER WIDERSTAND (kPa l-1 sec)
-5
0
5
10
15
20
25
30
35
40
45
50
55
60
0
3
6
9
12

FIG. 1

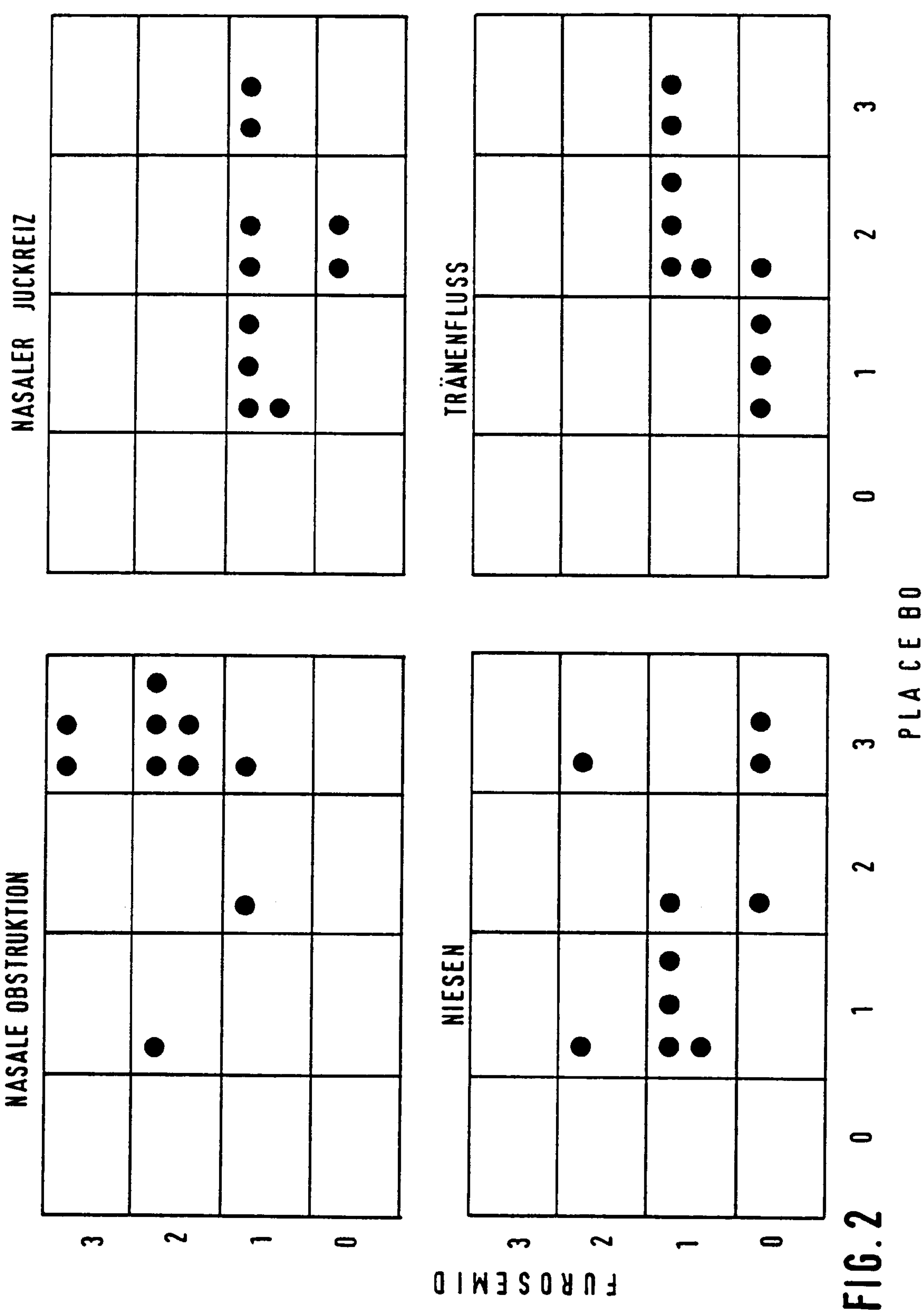
NASALE OBSTRUKTION
NASALER JUCKREIZ
NIESEN
TRÄNENFLUSS
PLACEBO
FUROSEMID
0
1
2
3

FIG. 2

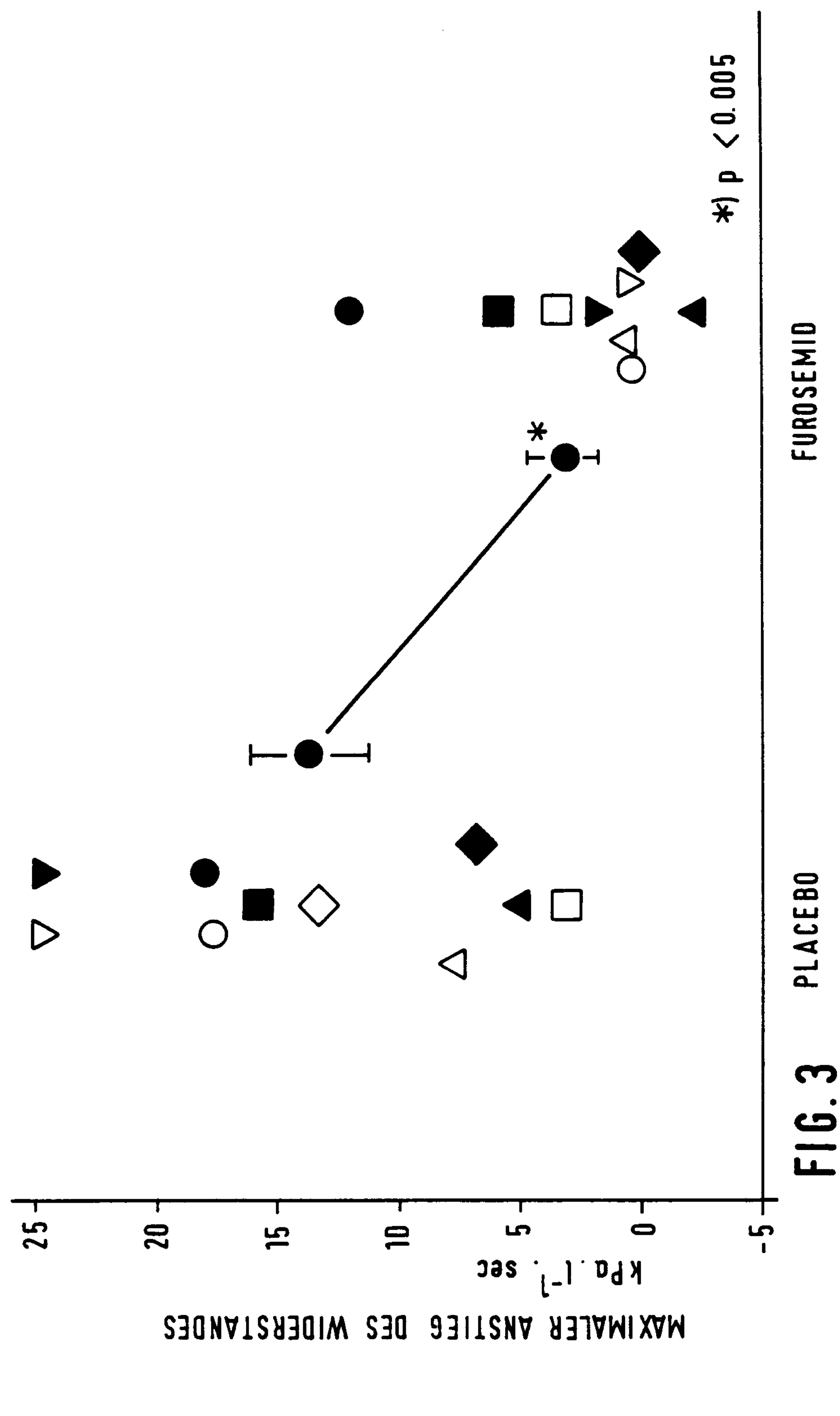
MAXIMALER ANSTIEG DES WIDERSTANDES
kPa . l⁻¹ . sec
25
20
15
10
5
0
-5
PLACEBO
FUROSEMID
*) p < 0.005

FIG. 3

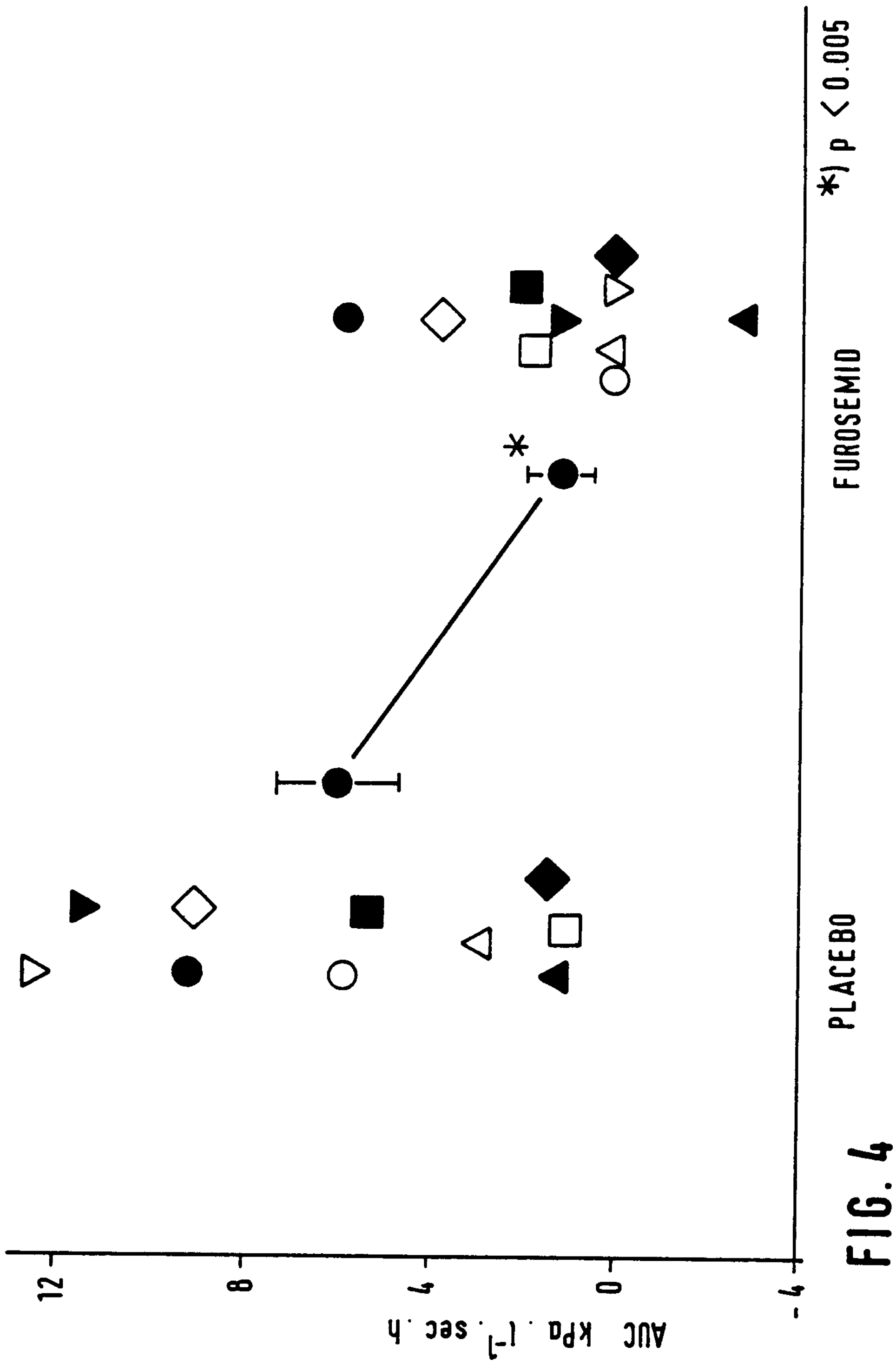


FIG. 4

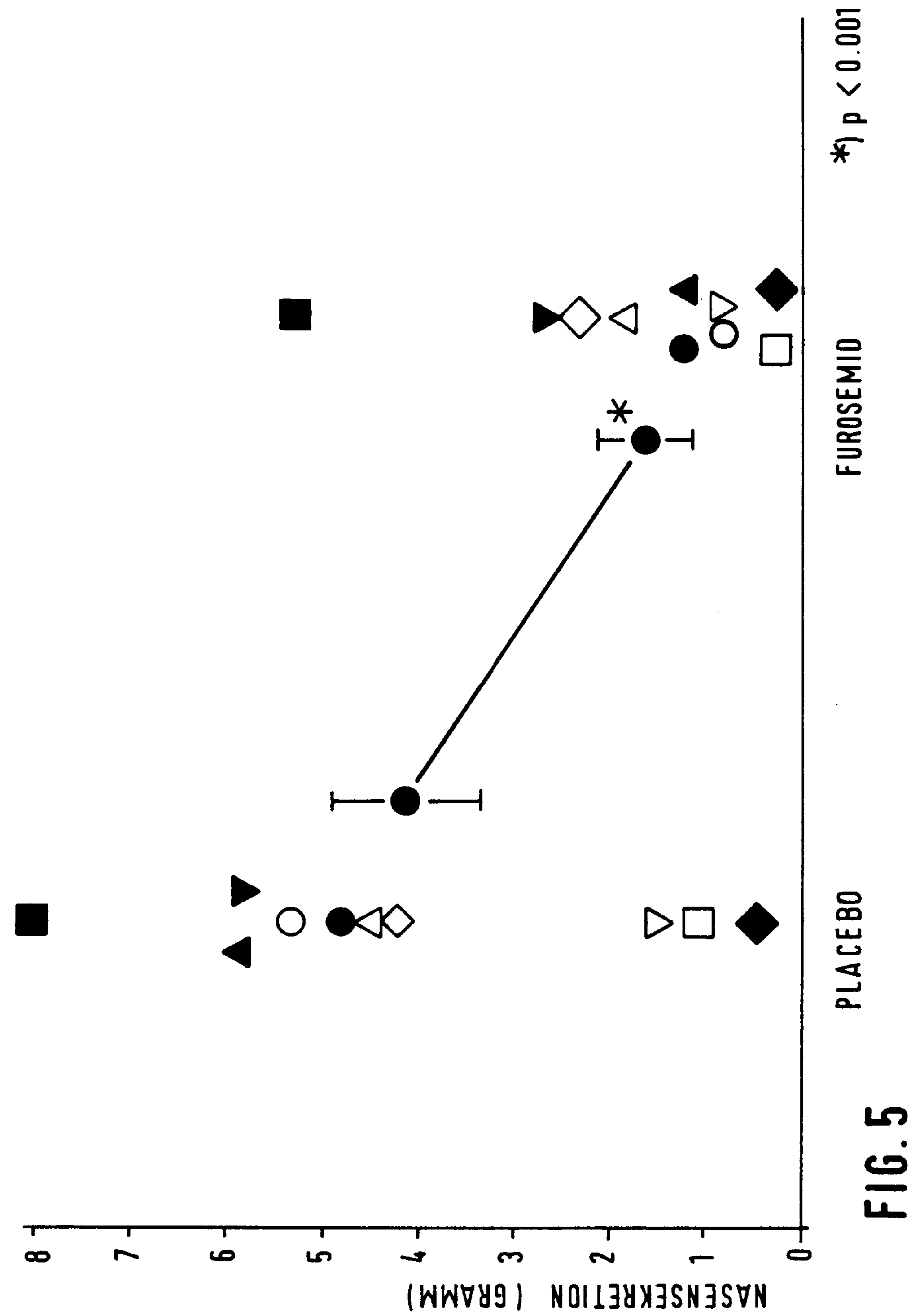


FIG. 5